# EUROPEAN PATENT APPLICATION

(11) **EP 4 013 050 A1**
(43) Date of publication of application: **15.06.2022**
(21) Application number: 20850257.5
(22) Date of filing: 22.07.2020
(51) Int. Cl.: H04N 13/239, A61B 1/00, A61B 90/20, G02B 21/20, G02B 21/22, G02B 21/36, H04N 13/296, H04N 13/332

(54) **SURGERY IMAGING SYSTEM, SIGNAL PROCESSING DEVICE, AND SIGNAL PROCESSING METHOD**

(30) Priority: 05.08.2019 JP 2019143718
(71) Applicant: Sony Group Corporation, Tokyo 108-0075 (JP)
(72) Inventor: KATSUKI, Shinji, Tokyo 108-0075 (JP)
(74) Representative: D Young & Co LLP
(86) International application number: PCT/JP2020/028388
(87) International publication number: WO 2021/024804

(57) **Abstract**

The present technology relates to a surgical image-capturing system, a signal processing device, and a signal processing method that enable improvement in the image quality of a 3D image of a surgical site.

Provided is a surgical image-capturing system including: a first image-capturing unit configured to capture a surgical site and output a first image-capturing signal; a second image-capturing unit configured to capture the surgical site at an angle different from an angle of the first image-capturing unit in a frame cycle identical to a frame cycle of the first image-capturing unit and output a second image-capturing signal; an image-capturing control unit configured to control respective image-capturing timings of the first image-capturing unit and the second image-capturing unit; and a signal generation unit configured to generate a three-dimensional (3D) image signal on the basis of the first image-capturing signal and the second image-capturing signal, in which the image-capturing control unit performs control such that a difference is made by half the frame cycle between the respective image-capturing timings of the first image-capturing unit and the second image-capturing unit. The present technology is applicable to, for example, a 3D image-capturing system for surgery.

## Description

### TECHNICAL FIELD

The present technology relates to a surgical image-capturing system, a signal processing device, and a signal processing method, and particularly, to a surgical image-capturing system, a signal processing device, and a signal processing method that enable improvement in the image quality of a three-dimensional (3D) image of a surgical site.

### BACKGROUND ART

Conventionally, proposed has been display of a 3D image of a surgical site of a patient by use of an active 3D display system with a piece of active-shutter 3D eyewear (see, for example, Patent Document 1).

### CITATION LIST

### PATENT DOCUMENT

Patent Document 1: Japanese Patent Application Laid-Open No. 2018-152657

### SUMMARY OF THE INVENTION

### PROBLEMS TO BE SOLVED BY THE INVENTION

However, with such a piece of active 3D eyewear, images for the left eye and images for the right eye are alternately displayed in a time division manner. Thus, a difference in depth may occur due to movement of a subject, resulting in deterioration in the image quality.

The present technology has been made in view of such a situation, and an object of the present technology is to enable improvement in the image quality of a 3D image of a surgical site.

### SOLUTIONS TO PROBLEMS

According to a first aspect of the present technology, provided is a surgical image-capturing system including: a first image-capturing unit configured to capture a surgical site and output a first image-capturing signal; a second image-capturing unit configured to capture the surgical site at an angle different from an angle of the first image-capturing unit in a frame cycle identical to a frame cycle of the first image-capturing unit and output a second image-capturing signal; an image-capturing control unit configured to control respective image-capturing timings of the first image-capturing unit and the second image-capturing unit; and a signal generation unit configured to generate a 3D image signal on the basis of the first image-capturing signal and the second image-capturing signal, in which the image-capturing control unit performs control such that a difference is made by half the frame cycle between the respective image-capturing timings of the first image-capturing unit and the second image-capturing unit.

According to a second aspect of the present technology, provided is a signal processing device including: an image-capturing control unit configured to control respective image-capturing timings of a first image-capturing unit and a second image-capturing unit, the first image-capturing unit being configured to capture a surgical site and output a first image-capturing signal, the second image-capturing unit being configured to capture the surgical site at an angle different from an angle of the first image-capturing unit in a frame cycle identical to a frame cycle of the first image-capturing unit and output a second image-capturing signal; and a signal generation unit configured to generate a 3D image signal on the basis of the first image-capturing signal and the second image-capturing signal, in which the image-capturing control unit performs control such that a difference is made by half the frame cycle between the respective image-capturing timings of the first image-capturing unit and the second image-capturing unit.

According to the second aspect of the present technology, provided is a signal processing method including: performing control such that a difference is made by half a frame cycle between respective image-capturing timings of a first image-capturing unit and a second image-capturing unit, the first image-capturing unit being configured to capture a surgical site and output a first image-capturing signal, the second image-capturing unit being configured to capture the surgical site at an angle different from an angle of the first image-capturing unit in the frame cycle identical to the frame cycle of the first image-capturing unit and output a second image-capturing signal; and generating a 3D image signal on the basis of the first image-capturing signal and the second image-capturing signal.

In the first aspect of the present technology, a first image-capturing unit captures a surgical site and outputs a first image-capturing signal; a second image-capturing unit captures the surgical site at an angle different from an angle of the first image-capturing unit in a frame cycle identical to a frame cycle of the first image-capturing unit and outputs a second image-capturing signal; control is performed such that a difference is made by half the frame cycle between the respective image-capturing timings of the first image-capturing unit and the second image-capturing unit; and a 3D image signal is generated on the basis of the first image-capturing signal and the second image-capturing signal.

In the second aspect of the present technology, control is performed such that a difference is made by half a frame cycle between respective image-capturing timings of a first image-capturing unit and a second image-capturing unit, the first image-capturing unit being configured to capture a surgical site and output a first image-capturing signal, the second image-capturing unit being configured to capture the surgical site at an angle different from an angle of the first image-capturing unit in the frame cycle identical to the frame cycle of the first image-capturing unit and output a second image-capturing signal; and a 3D image signal is generated on the basis of the first image-capturing signal and the second image-capturing signal.

### BRIEF DESCRIPTION OF DRAWINGS

Fig. 1 schematically illustrates an exemplary configuration of a patterned-retarder 3D display system.
Fig. 2 illustrates an exemplary vertical field-of-view angle of a patterned-retarder display device.
Fig. 3 is a graph showing exemplary characteristics of the vertical field-of-view angle of the patterned-retarder display device.
Fig. 4 schematically illustrates an exemplary configuration of an active-retarder 3D display system.
Fig. 5 explanatorily illustrates an operation of the active-retarder 3D display system.
Fig. 6 explanatorily illustrates an operation of the active-retarder 3D display system.
Fig. 7 explanatorily illustrates a difference in depth of the active-retarder 3D display system.
Fig. 8 explanatorily illustrates the difference in depth of the active-retarder 3D display system.
Fig. 9 explanatorily illustrates the difference in depth of the active-retarder 3D display system.
Fig. 10 explanatorily illustrates the difference in depth of the active-retarder 3D display system.
Fig. 11 explanatorily illustrates a difference in depth of the active-retarder 3D display system.
Fig. 12 explanatorily illustrates the difference in depth of the active-retarder 3D display system.
Fig. 13 illustrates an exemplary configuration of a microsurgery system to which the present technology is applied.
Fig. 14 is a block diagram illustrating an exemplary configuration of an image-capturing function of the microsurgery system in Fig. 13.
Fig. 15 is an explanatory flowchart of image-capturing-timing setting processing of the microsurgery system.
Fig. 16 explanatorily illustrates a method of generating a 3D image signal in a case where a display device is of an active-retarder type.
Fig. 17 explanatorily illustrates a method of generating a 3D image signal in a case where a display device is of a patterned-retarder type.
Fig. 18 is a block diagram of an exemplary configuration of a computer.

### MODE FOR CARRYING OUT THE INVENTION

Hereinafter, modes for carrying out the present technology will be described. The description will be given in the following order.
1. Issues on Various Types of 3D Display Systems
2. Embodiments
3. Modifications
4. Others

### <<1. Issues on Various Types of 3D Display Systems>>

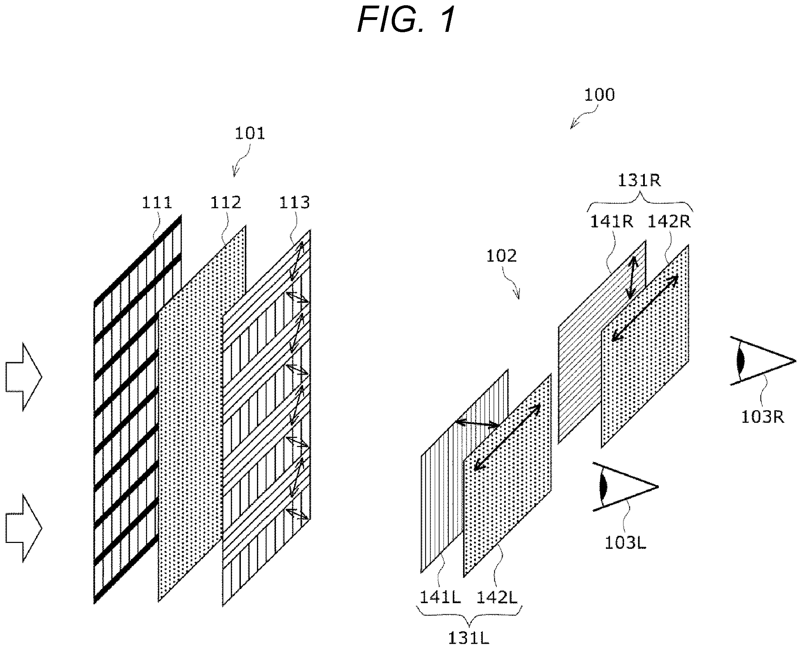

First, issues on various types of 3D display systems will be described with reference to Figs. 1 to 12.

### <Issues on Patterned-Retarder 3D Display System>

Fig. 1 schematically illustrates an exemplary configuration of a patterned-retarder 3D display system 100 that is one of passive 3D display systems.

The 3D display system 100 includes a display device 101 and a piece of polarization eyewear 102.

The display device 101 includes a display panel 111, a polarizing plate 112, and a patterned retarder 113, and the display panel 111, the polarizing plate 112, and the patterned retarder 113 are layered in this order from the backlight light source (not illustrated) side.

The display panel 111 displays a line-by-line 3D image with an image for the left eye (hereinafter, referred to as a left-eye image) and an image for the right eye (hereinafter, referred to as a right-eye image) alternately arranged for each pixel row (horizontal scanning line). For example, a left-eye image is displayed in the odd rows and a right-eye image is displayed in the even rows of the display panel 111.

The polarizing plate 112 is a polarizing plate of which the transmission axis agrees with the vertical direction of display panel 111.

The patterned retarder 113 is a retarder of which the retardation alternately changes for each pixel row (horizontal scanning line). For example, the retardation of the odd rows of the patterned retarder 113 is set to +λ/4 (λ is the wavelength to be used), and the retardation of the even rows is set to -λ/4.

Thus, the light for a right-eye image having entered into and passed through the odd rows of the display panel 111 from the backlight light source (hereinafter, referred to as right-eye image light) passes through the polarizing plate 112 and the odd rows of the patterned retarder 113, so that the right-eye image light is converted into clockwise circularly polarized light. The light for a left-eye image having entered into and passed through the even rows of the display panel 111 from the backlight light source (hereinafter, referred to as left-eye image light) passes through the polarizing plate 112 and the even rows of the patterned retarder 113, so that the left-eye image light is converted into counter-clockwise circularly polarized light.

The piece of polarization eyewear 102 includes a left-eye lens 131L and a right-eye lens 131R.

The left-eye lens 131L includes a retarder 141L and a polarizing plate 142L, and the retarder 141L and the polarizing plate 142L are layered in this order from the display device 101 side. The retardation of the retarder 141L is -λ/4, and the transmission axis of the polarizing plate 142L agrees with the horizontal direction of the display panel 111. Thus, the left-eye lens 131L has optical characteristics corresponding to the counter-clockwise circularly polarized light for the left-eye image of the display device 101.

The right-eye lens 131R includes a retarder 141R and a polarizing plate 142R, and the retarder 141R and the polarizing plate 142R are layered in this order from the display device 101 side. The retardation of the retarder 141R is +λ/4, and the transmission axis of the polarizing plate 142R agrees with the horizontal direction of the display panel 111. Thus, the right-eye lens 131R has optical characteristics corresponding to the clockwise circularly polarized light for the right-eye image of the display device 101.

Therefore, the left-eye image light having converted in the counter-clockwise circularly polarized light output from the display device 101 passes through the left-eye lens 131L and enters into the left eye 103L of the user, while being blocked by the polarizing plate 142R of the right-eye lens 131R and not entering into the right eye 103R. In contrast, the right-eye image light having converted in the clockwise circularly polarized light output from the display device 101 passes through the right-eye lens 131R and enters into the right eye 103R of the user, while being blocked by the polarizing plate 142L of the left-eye lens 131L and not entering into the left eye 103L.

As above, when the user views the three-dimensional image displayed by the display device 101 through the piece of polarization eyewear 102, the user visually recognizes the left-eye image with the left eye 103L and the right-eye image with the right eye 103R, resulting in achievement of a stereoscopic view.

However, in the patterned-retarder 3D display system 100, a phenomenon so-called crosstalk may occur depending on the position of the user to the display device 101. That is, due to entry of the right-eye image light into the left eye 103L and entry of the left-eye image light into the right eye 103R, the right-eye image may be visually recognized with the left eye 103L, and the left-eye image may be visually recognized with the right eye 103R.

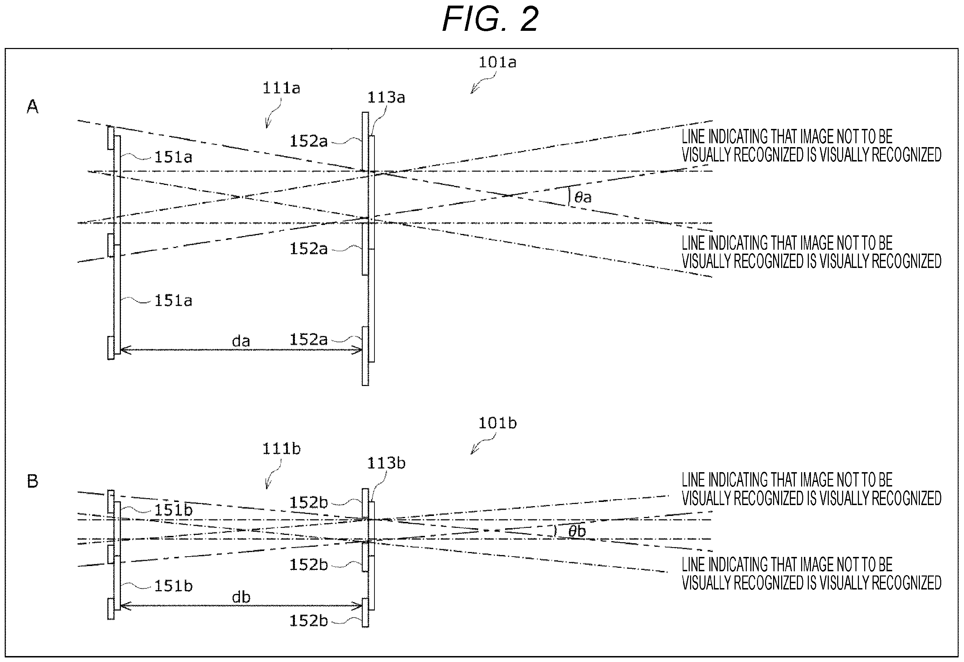

A of Fig. 2 schematically illustrates an enlarged side face of a display device 101a having a full high definition (FHD) resolution. Note that a polarizing plate 112 is not illustrated in A of Fig. 2.

As illustrated in A of Fig. 2, it is likely that from a beam splitter 152a provided on a patterned retarder 113a ahead of a certain pixel 151a, in addition to the light from the pixel 151a, light comes out from another pixel 151a vertically adjacent to the pixel 151a. Then, outside the vertical field-of-view angle θa, the respective images not to be visually recognized with the left and right eyes are visually recognized with the left and right eyes, resulting in blur of the contour of an object or impairment in the stereoscopic effect.

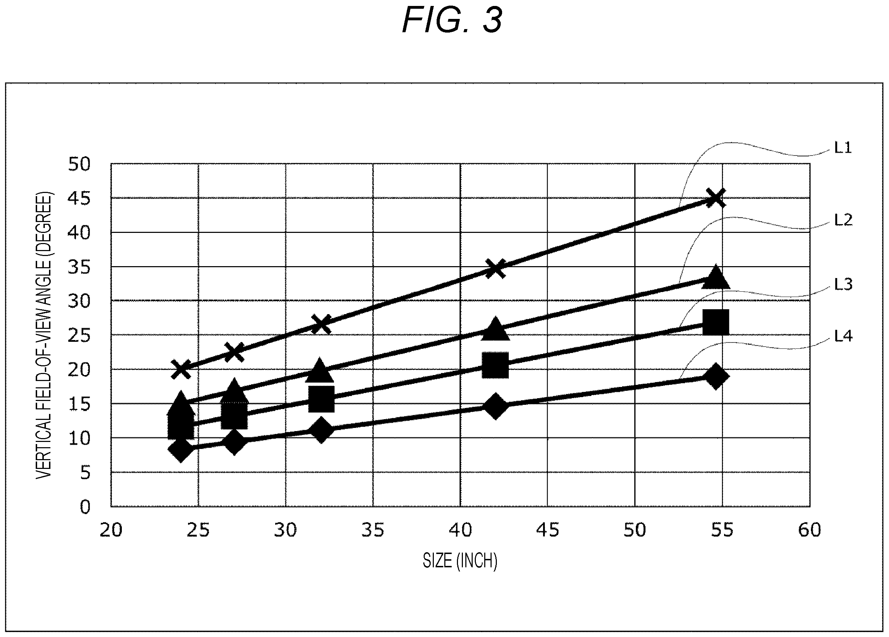

Fig. 3 is a graph showing exemplary characteristics of the vertical field-of-view angle θa of the display device 101a in A of Fig. 2. In Fig. 3, the horizontal axis represents the size (unit: inch) of a display panel 111a of the display device 101a, and the vertical axis represents the vertical field-of-view angle (unit: degree). A line L1 indicates the characteristic of the vertical field-of-view angle θ for the glass thickness da = 0.1 mm of the display panel 111a. A line L2 indicates the characteristic of the vertical field-of-view angle θ for the glass thickness da = 0.2 mm. A line L3 indicates the characteristic of the vertical field-of-view angle θ for the glass thickness da = 0.3 mm. A line L4 indicates the characteristic of the vertical field-of-view angle θ for the glass thickness da = 0.5 mm.

As shown in this graph, as the size of the display panel 111a decreases, the vertical field-of-view angle θa decreases, so that crosstalk is likely to occur.
Further, as the glass thickness of the display panel 111a increases, the vertical field-of-view angle θa decreases, so that crosstalk is likely to occur.

In addition, B of Fig. 2 schematically illustrates an enlarged side face of a display device 101b having a 4K resolution. Note that a polarizing plate 112 is not illustrated in B of Fig. 2.

When the display device 101b in B of Fig. 2 is compared with the display device 101a in A of Fig. 2, the pitch of a pixel 151b and the pitch of a beam splitter 152b are narrower due to an increase in the resolution. However, the glass thickness db of a display panel 111b is substantially similar to the glass thickness da of the display panel 111a. As a result, the vertical field-of-view angle θb decreases and crosstalk is likely to occur.

As described above, in display device 101, as the resolution increases, as the size of display panel 111 decreases, or as the glass thickness of display panel 111 increases, the vertical field-of-view angle decreases and crosstalk is likely to occur.

In addition, due to a further increase in the resolution of the display device 101 in the future, it is assumed that the vertical field-of-view angle will further decrease. For this issue, for example, it is conceivable to reduce the glass thickness of the display panel 111. However, there is a limit to the reduction in the glass thickness of the display panel 111 in consideration of durability, productivity, and others.

Furthermore, due to further miniaturization of the display panel 111 resulting from an increase in the resolution of the display device 101, it is assumed that the requirement for the attachment accuracy of the patterned retarder 113 becomes strict and the productivity is lowered.

Still furthermore, the production scale and production facilities of the display panels each including a patterned retarder tend to be reduced due to, for example, low sales of 3D TVs for consumers. As a result, for example, the cost and risk for designing a new display panel and introducing a production facility for a medical 3D display device are increasing.

### <Problems on Active-Retarder 3D Display System>

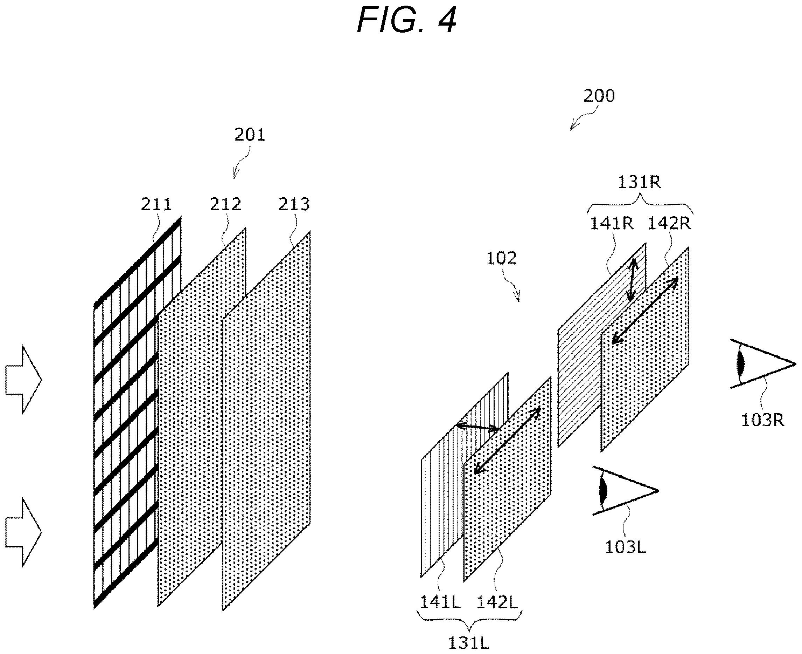

Fig. 4 schematically illustrates a configuration of an active-retarder 3D display system 200 that is one of passive 3D display systems. Note that, in the drawing, portions corresponding to those of the 3D display system 100 in Fig. 1 are denoted with the same reference signs, and thus the description thereof will not be given appropriately.

The 3D display system 200 is identical to the 3D display system 100 in that it includes a piece of polarization eyewear 102, and is different in that it includes a display device 201 instead of the display device 101.

The display device 201 includes a display panel 211, a polarizing plate 212, and an active retarder 213, and the display panel 211, the polarizing plate 212, and the active retarder 213 are layered in this order from the backlight light source (not illustrated) side.

The display panel 211 displays a frame-sequential 3D image in which left-eye images and right-eye images are alternately arranged in a time division manner.

Similarly to the polarizing plate 112 of display device 101, the polarizing plate 212 is a polarizing plate of which the transmission axis agrees with the vertical direction of display panel 211.

The active retarder 213 is disposed on the front face of the polarizing plate 212 and serves as a shutter mechanism together with the polarizing plate 212. The active retarder 213 has a retardation that is switched to +λ/4 or -λ/4 (λ is the wavelength to be used) due to application of an electric signal.

The display device 201 alternately displays the left-eye images and the right-eye images on the display panel 111 on a frame basis, and switches the retardation of the active retarder 213 in synchronization with image switching.

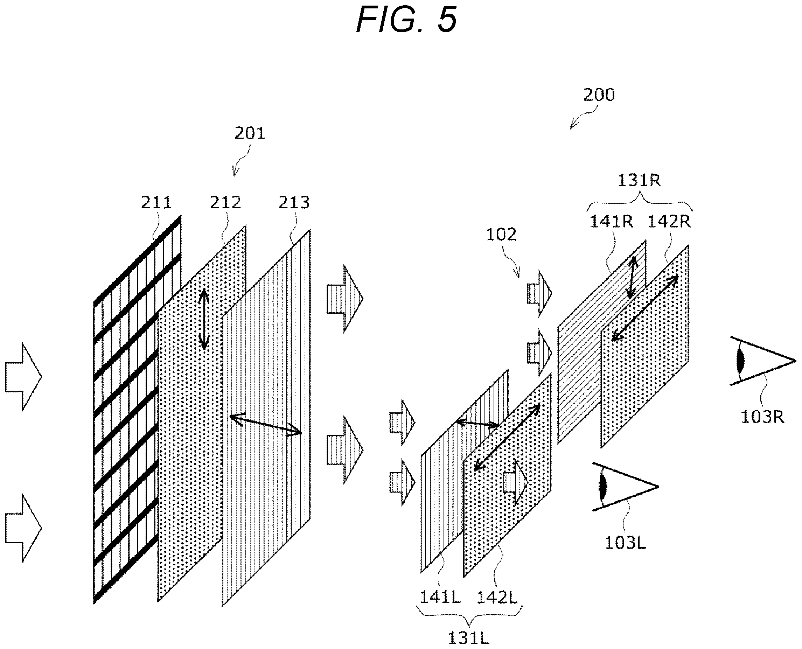

Specifically, as illustrated in Fig. 5, during display of such a left-eye image, the applied voltage to the active retarder 213 is on (Von), and the retardation of the active retarder 213 is set at -λ/4. With this arrangement, the light for the left-eye image having entered into and passed through the display panel 211 from the backlight light source (not illustrated) (hereinafter, referred to as left-eye image light) passes through the polarizing plate 212 and the active retarder 213, so that the left-eye image light is converted into counter-clockwise circularly polarized light. Thus, the left-eye image light having come out from the display device 201 passes through the left-eye lens 131L and enters into the left eye 103L of the user, while being blocked by the polarizing plate 142R of the right-eye lens 131R and not entering into the right eye 103R.

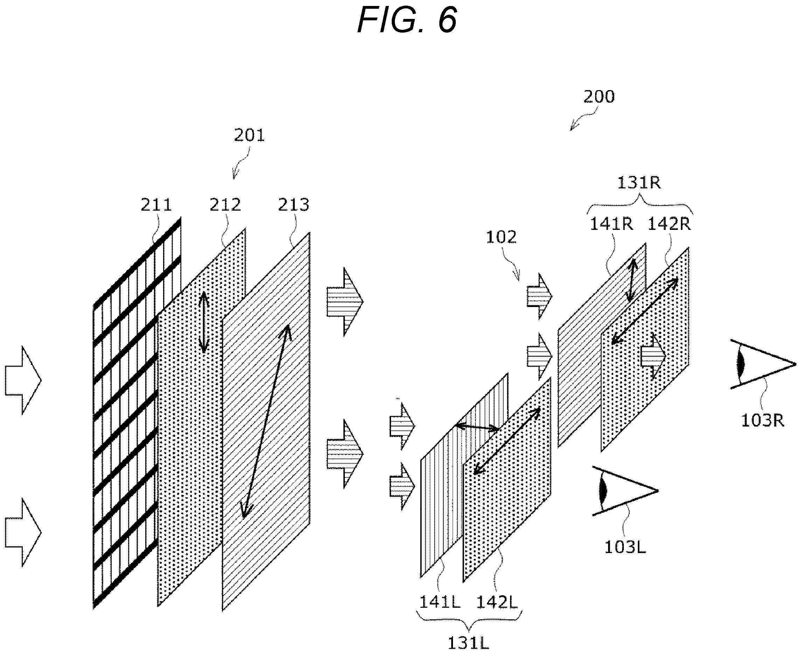

Further, as illustrated in Fig. 6, during display of such a right-eye image, the applied voltage to the active retarder 213 is off (Voff), and the retardation of the active retarder 213 is set at +λ/4. With this arrangement, the light for the right-eye image having entered into and passed through the display panel 211 from the backlight light source (not illustrated) (hereinafter, referred to as right-eye image light) passes through the polarizing plate 212 and the active retarder 213, so that the right-eye image light is converted into clockwise circularly polarized light. Thus, the right-eye image light having come out from the display device 201 passes through the right-eye lens 131R and enters into the right eye 103R of the user, while being blocked by the polarizing plate 142L of the left-eye lens 131L and not entering into the left eye 103L.

As above, when the user views the three-dimensional image displayed by the display device 201 through the piece of polarization eyewear 102, the user visually recognizes the left-eye image with the left eye 103L and the right-eye image with the right eye 103R, resulting in achievement of a stereoscopic view.

However, in the 3D display system 200, the left-eye image and the right-eye image are displayed in a time division manner. Thus, a difference in the amount of disparity recognized by the user may occur at display of a moving image, so that a difference in depth may occur.

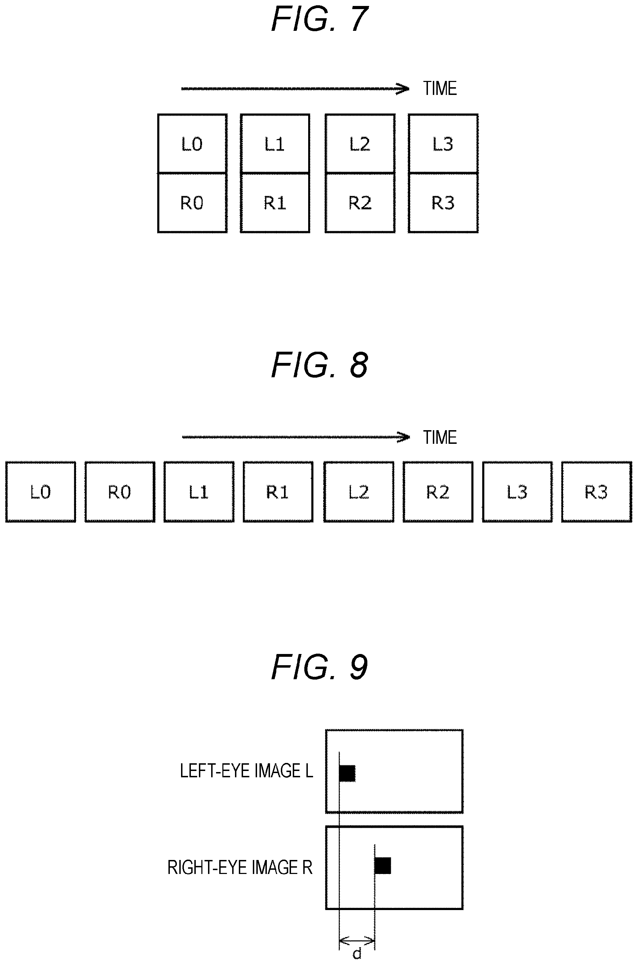

Specifically, Fig. 7 illustrates capturing or generation timing of a left-eye image and a right-eye image for a three-dimensional image to be input to the display device 201. Here, L represents a left-eye image, R represents a right-eye image, and the respective numbers attached to L and R indicate sequence numbers. Such a sequence number indicates a capturing order or a generation order of each image. For example, a left-eye image L0 and a right-eye image R0 are captured or generated at the same time, a left-eye image L1 and a right-eye image R1 are captured or generated at the same time, a left-eye image L2 and a right-eye image R2 are captured or generated at the same time, and a left-eye image L3 and a right-eye image R3 are captured or generated at the same time.

Note that hereinafter, the left-eye images L0, L1, L2, and L3... will be each simply referred to as a left-eye image L in a case where it is not necessary to individually distinguish them, and the right-eye images R0, R1, R2, and R3... will be each simply referred to as a right-eye image R in a case where it is not necessary to individually distinguish them.

In addition, as illustrated in Fig. 8, such left-eye images L and right-eye images R are alternately input into and displayed on the display device 201.

As a result, the left-eye image L and the right-eye image R that originally should be simultaneously visually recognized with the left and right eyes are visually recognized with a time difference of half a frame. For example, the right-eye image R0 is visually recognized with a delay of half a frame with respect to the left-eye image L0. Therefore, apparent disparity may vary depending on the movement direction and speed of a subject.

For example, in a case where the left-eye image L is displayed prior to the right-eye image R, described will be a case of display of a moving image in which the subject having a disparity of d pixels between the left-eye image L and the right-eye image R moves from left to right within the image at a speed of Δx pixels per frame cycle as illustrated in Fig. 9.

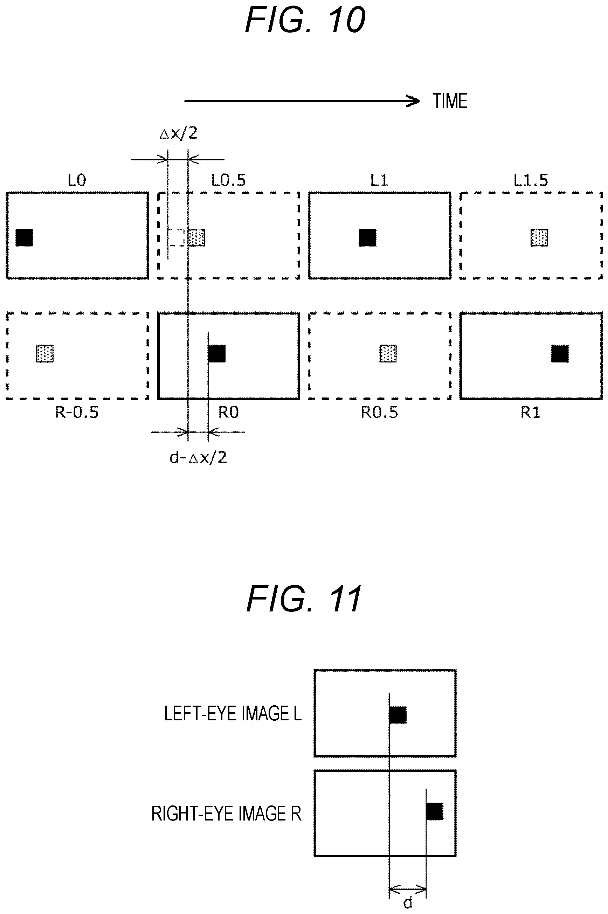

Fig. 10 illustrates the display of the moving image by rearrangement of L0-R0-L1-R1-L2-R2-... in this order as illustrated in Fig. 8.

As described above, the right-eye image R0 is displayed with a delay of half a frame with respect to the left-eye image L0, and the right-eye image R1 is displayed with a delay of half a frame with respect to the left-eye image L1. In addition, the subject moves right by Δx pixels between the left-eye image L0 and the left-eye image L1. Thus, at the time when the right-eye image R0 is displayed with a delay of half a frame with respect to the left-eye image L0, the subject moves right by Δx/2 pixels within a virtual left-eye image L0.5 between the left-eye image L0 and the left-eye image L1 in the user's brain.

In contrast, the subject within the right-eye image R0 displayed at the same time as the left-eye image L0.5 is on the right side by d pixels as compared with the left-eye image L0. The disparity, however, decreases by Δx/2 pixels between the right-eye image R0 and the left-eye image L0.5 that are recalled in the user's brain. Thus, the user feels that the subject is closer than it actually is.

Further, for example, described will be a case of display of a moving image in which the subject having a disparity of d pixels between the left-eye image L and the right-eye image R moves from right to left within the image at a speed of Δx pixels per frame cycle as illustrated in Fig. 11.

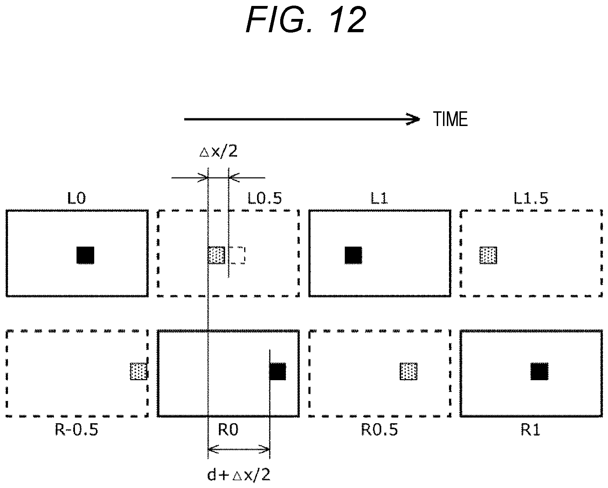

Fig. 12 illustrates the display of the moving image by rearrangement of L0-R0-L1-R1-L2-R2-... in this order as illustrated in Fig. 8.

As described above, the right-eye image R0 is displayed with a delay of half a frame with respect to the left-eye image L0, and the right-eye image R1 is displayed with a delay of half a frame with respect to the left-eye image L1. In addition, the subject moves left by Δx pixels between the left-eye image L0 and the left-eye image L1. Thus, at the time when the right-eye image R0 is displayed with a delay of half a frame with respect to the left-eye image L0, the subject moves left by Δx/2 pixels within the virtual left-eye image L0.5 between the left-eye image L0 and the left-eye image L1 in the user's brain.

In contrast, the subject within the right-eye image R0 displayed at the same time as the left-eye image L0.5 is on the left side by d pixels as compared with the left-eye image L0. The disparity, however, increases by Δx/2 pixels between the right-eye image R0 and the left-eye image L0.5 that are recalled in the user's brain. Thus, the user feels that the subject is farther than it actually is.

For example, in a case where the 3D display system 200 is used as a monitor of a surgery room, if a phenomenon in which the anteroposterior relationship of the subject is reversed occurs due to such a difference in depth, there is a possibility that a fatal mistake such as damaging an organ as the subject occurs.

For this issue, for example, in order to reduce the difference in depth, it is assumed that an interpolation image for the right eye corresponding to the left-eye image L0.5 is generated and displayed on the basis of the right-eye image R0 and the right-eye image R1.

However, for an image for surgery, it is important to correctly express an entity rather than easiness of viewing or beauty of appearance, and thus it is not generally accepted to use an interpolation image. In addition, in order to generate an interpolation image, it is necessary to wait for capturing of a right-eye image after one frame, so that delay occurs in image display. This may be a fatal problem at a surgical location where a real-time determination is required.

### <Issues on Active 3D Display System>

In an active 3D display system, left-eye images and right-eye images are alternately displayed in a time division manner, similarly to the active-retarder 3D display system 200. Thus, similarly to the 3D display system 200, a difference in depth may occur.

In addition, conventionally, a passive 3D display system with the piece of polarization eyewear 102 described above is mainly used as a monitor of a 3D image for surgery. However, a piece of active-shutter 3D eyewear used with an active display device is not compatible with the piece of polarization eyewear 102 used with a passive display device. Thus, in a case where a plurality of display devices is provided in a surgery room, all the display devices need to be unified to the active type, or the user needs to change a piece of eyewear depending on the display devices.

Furthermore, in a case where a plurality of active display devices is provided in a surgery room and a plurality of users view each display device, it is necessary to synchronize each display device with the piece of 3D eyewear of each user, resulting in complication of the control.

### <<2. Embodiments>>

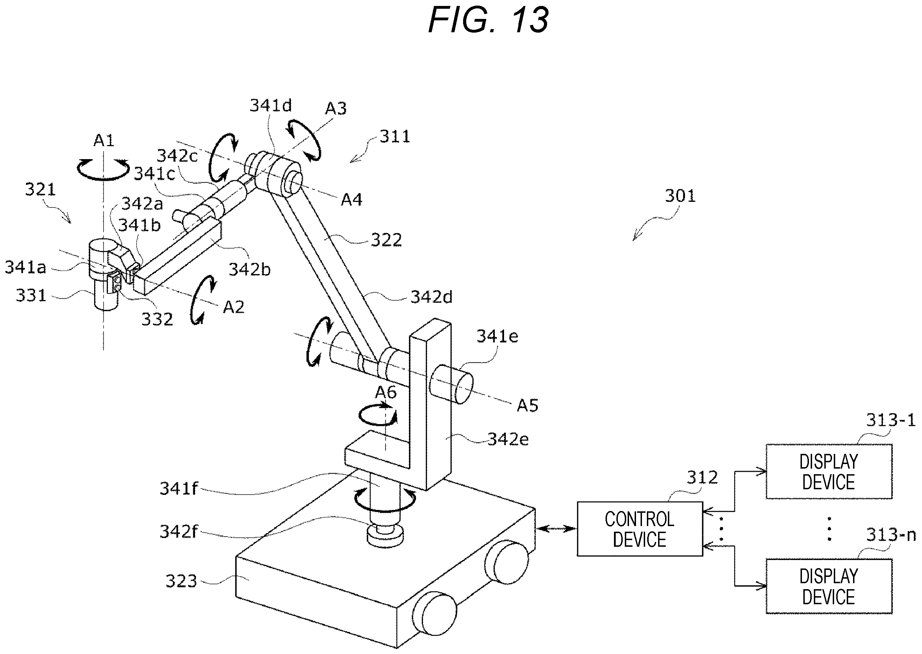

Next, embodiments of the present technology will be described with reference to Figs. 13 to 17.

### <Exemplary Configuration of Microsurgery System 301>

Fig. 13 illustrates an exemplary configuration of a microsurgery system 301 to which the present technology is applied.

The microsurgery system 301 includes a microscope device 311, a control device 312, and display devices 313-1 to 313-n. The microscope device 311 and the control device 312 serve as a surgical image-capturing system.

Note that, hereinafter, the display devices 313-1 to 313-n will be each simply referred to as a display device 313 in a case where it is not necessary to individually distinguish them.

The microscope device 311 includes a microscope 321 for enlargement observation of an observation target (surgical site of a patient), an arm 322 having a distal end supporting the microscope 321, and a base 323 supporting the proximal end of the arm 322.

The microscope 321 includes a tubular portion 331 having a substantially cylindrical shape, an image-capturing unit (not illustrated) provided inside the tubular portion 331, and an operation unit 332 provided in a partial region of the outer periphery of the tubular portion 331. The microscope 321 is an electronic image-capturing microscope (video microscope) that electronically captures an image as a capturing target with the image-capturing unit.

The tubular portion 331 has an opening face at the lower end thereof, and the opening face is provided with a cover glass for protecting the image-capturing unit inside the tubular portion 331. Light from the observation target (hereinafter, also referred to as observation light) passes through the cover glass and enters into the image-capturing unit inside the tubular portion 331. Note that a light source including, for example, a light emitting diode (LED) may be provided inside the tubular portion 331, and the observation target may be irradiated with light from the light source though the cover glass during capturing an image.

The image-capturing unit includes an optical system that condenses the observation light and an image-capturing element that receives the observation light condensed by the optical system. The optical system includes a combination of a plurality of lenses including a zoom lens and a focus lens, and its optical characteristics are adjusted such that the observation light forms an image on the light-receiving face of the image-capturing element. The image-capturing element receives and photoelectrically converts the observation light to generate a signal corresponding to the observation light, that is, an image signal corresponding to the observation image. The image-capturing element includes, for example, any type of image sensor such as a complementary metal oxide semiconductor (CMOS) image sensor or a charge coupled device (CCD) image sensor.

Further, the image-capturing unit includes a drive mechanism that moves the zoom lens and the focus lens of the optical system along the optical axis. Appropriate movement of the zoom lens and the focus lens by the drive mechanism can adjust the magnification of an image as a capturing target and the focal length at the time of capturing the image. Furthermore, the image-capturing unit may be equipped with various functions that can be typically provided in an electronic image-capturing microscope, such as an auto exposure (AE) function and an auto focus (AF) function.

Note that, as described later, the microscope device 311 includes two image-capturing units that each capture a corresponding left-eye image or right-eye image corresponding to a stereoscopic view (3D display). The 3D display enables the surgeon to grasp more accurately the depth of a surgical site.

The operation unit 332 includes, for example, a cross lever, a switch or the like, and serves as an input means that receives an operation input from the user.
For example, the user can input an instruction for change of the magnification and the focal length (focus) of the observation image through the operation unit 332. The drive mechanism of each image-capturing unit appropriately moves the zoom lens and the focus lens in accordance with the instruction, so that the magnification and the focus can be adjusted.

The arm 322 includes a plurality of links (first link 342a to sixth link 342f) and a plurality of joints (first joint 341a to sixth joint 341f) that are turnably linked with each other.

The first joint 341a has a substantially columnar shape, and has a distal end (lower end) turnably supporting the upper end of the tubular portion 331 of the microscope 321 about a rotation axis (first axis A1) parallel to the central axis of the tubular portion 331. Here, the first joint 341a can be provided such that the first axis A1 agrees with the optical axis of each image-capturing unit of the microscope 321. With this arrangement, turning of the microscope 321 about the first axis A1 enables change of the field of view such that the image as the capturing target rotates.

The first link 342a has a distal end fixedly supporting the first joint 341a. Specifically, the first link 342a is a rod-shaped member having a substantially L-shape and one side on its distal end side extends in a direction orthogonal to the first axis A1. The first link 342a is connected to the first joint 341a such that the end portion of the one side abuts on the upper end portion of the outer periphery of the first joint 341a. The second joint 341b is connected to the end portion of the other side on the proximal end side of the substantially L-shape of the first link 342a.

The second joint 341b has a substantially columnar shape, and has a distal end turnably supporting the proximal end of the first link 342a about a rotation axis (second axis A2) orthogonal to the first axis A1. The distal end of the second link 342b is fixedly connected to the proximal end of the second joint 341b.

The second link 342b is a rod-shaped member having a substantially L-shape and one side on its distal end side extends in a direction orthogonal to the second axis A2. The end portion of the one side is fixedly connected to the proximal end of the second joint 341b. The third joint 341c is connected to the other side on the proximal end side of the substantially L-shape of the second link 342b.

The third joint 341c has a substantially columnar shape, and has a distal end turnably supporting the proximal end of the second link 342b about a rotation axis (third axis A3) orthogonal to the first axis A1 and the second axis A2. The distal end of the third link 342c is fixedly connected to the proximal end of the third joint 341c. Turning of the components on the distal end side including the microscope 321 about the second axis A2 and the third axis A3 allows movement of the microscope 321 such that the position of the microscope 321 is changed in the horizontal plane. That is, control of the rotation about the second axis A2 and the third axis A3 enables the field of view of the image as the capturing target to be moved in a plane.

The third link 342c has a substantially columnar shape on its distal end side, and the proximal end of the third joint 341c is fixedly connected to the distal end of the columnar shape such that both have substantially the same central axis. The third link 342c has a prismatic shape on the proximal end side, and the fourth joint 341d is connected to the end portion thereof.

The fourth joint 341d has a substantially columnar shape, and has a distal end turnably supporting the proximal end of the third link 342c about a rotation axis (fourth axis A4) orthogonal to the third axis A3. The distal end of the fourth link 342d is fixedly connected to the proximal end of the fourth joint 341d.

The fourth link 342d is a rod-shaped member extending substantially linearly and extends orthogonally to the fourth axis A4. The fourth link 342d is fixedly connected to the fourth joint 341d such that the end portion of its distal end abuts on a side face of the substantially columnar shape of the fourth joint 341d. The fifth joint 341e is connected to the proximal end of the fourth link 342d.

The fifth joint 341e has a substantially columnar shape, and on its distal end side, turnably supports the proximal end of the fourth link 342d about a rotation axis (fifth axis A5) parallel to the fourth axis A4. The distal end of the fifth link 342e is fixedly connected to the proximal end of the fifth joint 341e. The fourth axis A4 and the fifth axis A5 are rotation axes that allow upward and downward movement of the microscope 321. Turning of the components on the distal end side including the microscope 321 about the fourth axis A4 and the fifth axis A5 allows adjustment of the height of the microscope 321, that is, the distance between the microscope 321 and the observation target.

The fifth link 342e has a combination of a first member having a substantially L-shape and having one side perpendicularly extending and the other side horizontally extending, and a rod-shaped second member extending perpendicularly downward from the portion horizontally extending of the first member. The proximal end of the fifth joint 341e is fixedly connected to the vicinity of the upper end of the portion perpendicularly extending of the first member of the fifth link 342e. The sixth joint 341f is connected to the proximal end (lower end) of the second member of the fifth link 342e.

The sixth joint 341f has a substantially columnar shape, and on its distal end side, turnably supports the proximal end of the fifth link 342e about a rotation axis (sixth axis A6) parallel to the vertical direction. The distal end of the sixth link 342f is fixedly connected to the proximal end of the sixth joint 341f.

The sixth link 342f is a rod-shaped member perpendicularly extending, and has a proximal end fixedly connected to the upper face of the base 323.

The respective rotatable ranges of the first joint 341a to the sixth joint 341f are set appropriately so as to allow desired movement of the microscope 321. This arrangement can achieve movement of a total of six degrees of freedom, that is, three degrees of freedom in translation and three degrees of freedom in rotation regarding movement of the microscope 321, at the arm 322 having the above configuration. Thus, the configuration of the arm 322 so as to achieve the six degrees of freedom regarding the movement of the microscope 321 enables free control of the position and posture of the microscope 321 within the movable range of the arm 322. Therefore, the surgical site can be observed at any angle, so that the surgery can be performed more smoothly.

Note that the illustrated configuration of the arm 322 is merely an example, and thus the arm 322 may be appropriately designed in the number and shape (length) of links, the number of joints, arrangement positions, the directions of rotation axes, and others, for achievement of a desired degree of freedom.

Here, the first joint 341a to the sixth joint 341f are each provided with an actuator equipped with, for example, a drive mechanism such as a motor, an encoder that detects the rotation angle of the corresponding joint. In addition, the control device 312 appropriately controls the drive of each actuator provided in the first joint 341a to the sixth joint 341f, so that the posture of the arm 322, that is, the position and posture of the microscope 321 can be controlled.

Specifically, the control device 312 can grasp the current posture of the arm 322 and the current position and posture of the microscope 321 on the basis of information regarding the rotation angle of each joint detected by the corresponding encoder. The control device 312 calculates, with these pieces of grasped information, a control value (for example, rotation angle or torque to be generated) for each joint for realization of movement of the microscope 321 in response to an operation input from the user, and drives the drive mechanism of each joint in accordance with the control value. Note that, at this time, a method of controlling the arm 322 by the control device 312 is not limited, and thus various known control methods such as force control or position control may be applied.

The control device 312 includes, for example, some of a processor such as central processing unit (CPU) or a graphics processing unit (GPU), a control board mixedly equipped with a processor and a storage element such as a memory, a camera control unit (CCU), and the like. The control device 312 controls the respective operations of the microscope device 311 and the display device 313 to integrally control the operation of the microsurgery system 301.

For example, the control device 312 causes the actuators of the first joint 341a to the sixth joint 341f to operate in accordance with a predetermined control method to control the drive of the arm 322.

Further, for example, the control device 312 controls the image-capturing units of the microscope 321 of the microscope device 311 to control image-capturing processing of the surgical site of the patient.

Furthermore, for example, the control device 312 performs various types of signal processing on an image-capturing signal acquired by each image-capturing unit of the microscope 321 of the microscope device 311, generates a 3D image signal for display, and outputs the 3D image signal to each display device 313. In the signal processing, for example, performed are various types of signal processing such as development processing (demosaicing), high-image-quality processing (band emphasis processing, super-resolution processing, noise reduction (NR) processing, blur correction processing, and/or the like), enlargement processing (that is, electronic zoom processing), and 3D-image generation processing.

Note that the communication between the control device 312 and the microscope 321 and the communication between the control device 312 and the first joint 341a to the sixth joint 341f may be wired communication or wireless communication.

Note that, in this example, the control device 312 is provided as a device separate from the microscope device 311; however, the control device 312 may be installed inside the base 323 of the microscope device 311 and integrally formed with the microscope device 311. Alternatively, the control device 312 may include a plurality of devices. For example, a microcomputer, a control board, and others may be disposed in each of the microscope 321 and the first joint 341a to the sixth joint 341f of the arm 322, and these may be communicably connected to each other to achieve functions similar to those of the control device 312.

Each display device 313 includes a patterned-retarder display device (for example, the display device 101 of Fig. 1) or an active-retarder display device (for example, the display device 201 of Fig. 4). Each display device 313 is provided in a surgery room and displays a 3D image of the surgical site of the patient captured by the microscope 321 under the control by the control device 312.

Note that the display type of each display device 313 may be unified to either the patterned-retarder type or the active-retarder type, or may not be unified. In addition, each display device 313 may supply a signal indicating its display type to the control device 312. Furthermore, a plurality of display devices 313 is not necessarily provided, and a single display device may be provided.

### <Exemplary Configuration of Image-Capturing Function of Microsurgery System 301>

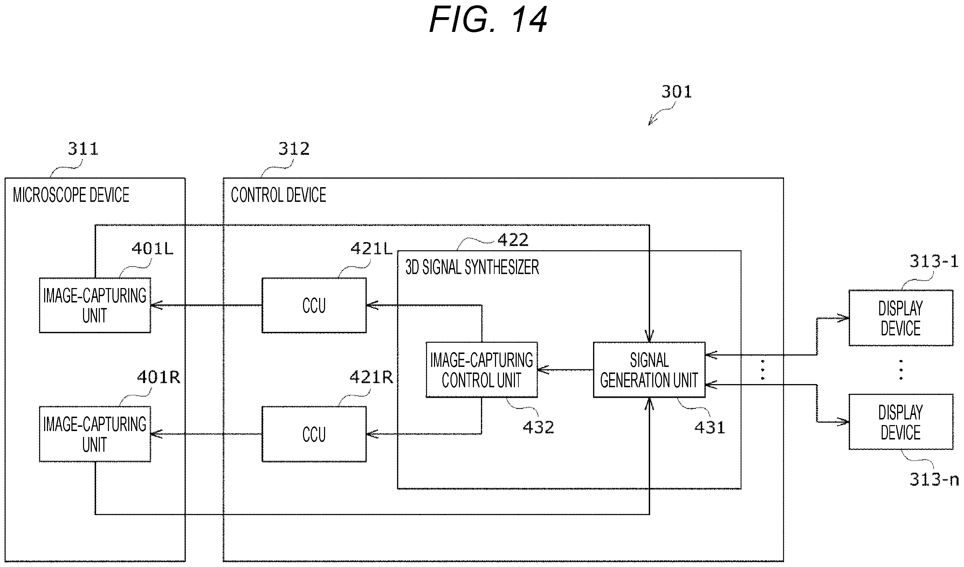

Fig. 14 illustrates an exemplary configuration of an image-capturing function of the microsurgery system 301 in Fig. 13.

The microscope device 311 includes an image-capturing unit 401L and an image-capturing unit 401R.

The control device 312 includes a CCU 421L and a CCU 421R, and a 3D signal synthesizer 422. The 3D signal synthesizer 422 includes a signal generation unit 431 and an image-capturing control unit 432.

Under the control by the CCU 421L, the image-capturing unit 401L captures a left-eye image visually recognized by the left eye of the user. The image-capturing unit 401L generates an image-capturing signal including the left-eye image (hereinafter, referred to as a left-eye image-capturing signal), and supplies the image-capturing signal to the signal generation unit 431.

Under the control by the CCU 421R, the image-capturing unit 401R captures a right-eye image visually recognized by the right eye of the user, at an angle different from that of the image-capturing unit 401L in the same frame cycle as the image-capturing unit 401. The image-capturing unit 401R generates an image-capturing signal including the right-eye image (hereinafter, referred to as a right-eye image-capturing signal), and supplies the image-capturing signal to the signal generation unit 431.

The CCU 421L controls the image-capturing unit 401L. For example, the CCU 421L controls the image-capturing timing of the image-capturing unit 401L on the basis of a left-image-capturing control signal supplied from the image-capturing control unit 432.

The CCU 421R controls the image-capturing unit 401R. For example, the CCU 421R controls the image-capturing timing of the image-capturing unit 401R on the basis of a right-image-capturing control signal supplied from the image-capturing control unit 432.

The signal generation unit 431 detects the display type (patterned-retarder type or active-retarder type) of a display device 313 and notifies the image-capturing control unit 432 of the detection result. Further, the signal generation unit 431 generates a 3D image corresponding to the display type of the display device 313 on the basis of the left-eye image-capturing signal and the right-eye image-capturing signal, and generates a 3D image signal including the generated 3D image. The signal generation unit 431 outputs the 3D image signal to each display device 313. Further, the signal generation unit 431 generates a reference clock signal as a reference of the operation of the control device 312, and supplies the reference clock signal to the image-capturing control unit 432.

The image-capturing control unit 432 controls the respective image-capturing timings of the image-capturing unit 401L and the image-capturing unit 401R on the basis of the display type of the display device 313. Specifically, on the basis of the display type of the display device 313, the image-capturing control unit 432 generates a left-image-capturing control signal for controlling the image-capturing timing of the image-capturing unit 401L and a right-image-capturing control signal for controlling the image-capturing timing of the image-capturing unit 401R. The image-capturing control unit 432 supplies the left-image-capturing control signal to the CCU 421L and supplies the right-image-capturing control signal to the CCU 421R.

### <Image-Capturing-Timing Setting Processing>

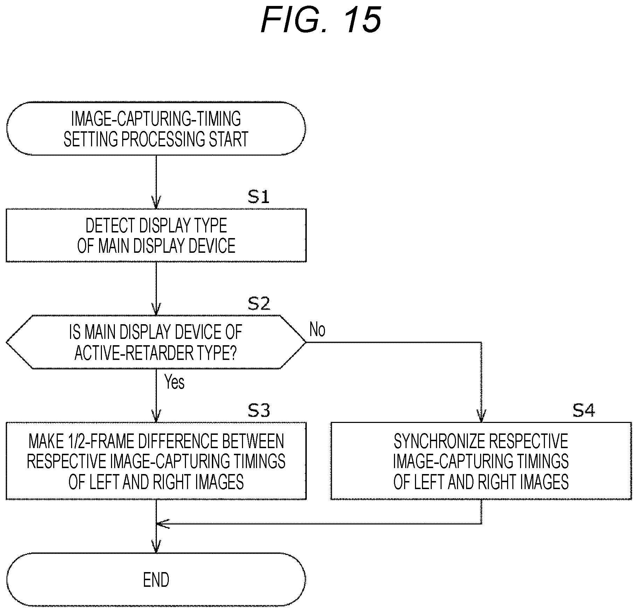

Next, image-capturing-timing setting processing executed by the microsurgery system 301 will be described with reference to the flowchart of Fig. 15.

Note that this processing is performed, for example, at the start of capturing of a surgical site by the microsurgery system 301.

In step S1, the signal generation unit 431 detects the display type of the main display device 313. Here, the main display device 313 is, for example, the display device 313 (for the main surgeon) set to be viewed by the main surgeon (for example, a surgical operator).

Note that a method of detecting the display type is not particularly limited.

For example, the signal generation unit 431 detects the display type of the main display device 313 on the basis of a signal indicating the display type output from the main display device 313.

Alternatively, for example, the user inputs the display type of the main display device 313 through the input unit (not illustrated) of the control device 312. The signal generation unit 431 detects the display type of the main display device 313 on the basis of the input from the user.

The signal generation unit 431 notifies the image-capturing control unit 432 of the display type of the main display device 313.

In step S2, the image-capturing control unit 432 determines whether or not the main display device 313 is of the active-retarder type. In a case where it is determined that the main display device 313 is of the active-retarder type, the processing proceeds to step S3.

In step S3, the microsurgery system 301 makes a 1/2-frame difference between the respective image-capturing timings of the left and right images.

Specifically, the signal generation unit 431 starts processing of generating a reference clock signal of 120 Hz and supplying the reference clock signal to the image-capturing control unit 432, for example.

The image-capturing control unit 432 starts processing of generating a left-image-capturing control signal and a right-image-capturing control signal on the basis of the reference clock signal, supplying the left-image-capturing control signal to the CCU 421L, and supplying the right-image-capturing control signal to the CCU 421R.

The left-image-capturing control signal and the right-image-capturing control signal include, respectively, for example, information indicating the timing at which the CCU 421L outputs a vertical synchronization signal and information indicating the timing at which the CCU 421R outputs a vertical synchronization signal. Then, the image-capturing control unit 432 performs control such that a difference is made by a 1/2 frame cycle (half a frame cycle) between the timing at which the vertical synchronization signal is output from the CCU 421L and the timing at which the vertical synchronization signal is output from the CCU 421R. That is, the image-capturing control unit 432 performs control such that the time difference between the timing at which the vertical synchronization signal is output from the CCU 421L and the timing at which the vertical synchronization signal is output from the CCU 421R is a 1/2 frame cycle.

Here, one frame cycle is the duration from the start of capturing an image to the start of capturing a subsequent image.

For example, the CCU 421L generates a vertical synchronization signal of 60 Hz and outputs the signal to the image-capturing unit 401L. On the other hand, the CCU 421R generates a vertical synchronization signal of 60 Hz, and outputs the signal to the image-capturing unit 401R at the timing with a 1/2 frame-cycle difference from the timing of the CCU 421L.

In accordance with the vertical synchronization signal from the CCU 421L, the image-capturing unit 401L starts processing of capturing a left-eye image with a 4K resolution and a frame rate of 60P, for example.
Further, the image-capturing unit 401L starts processing of generating a left-eye image-capturing signal including the captured left-eye image and supplying the signal to the signal generation unit 431.

In accordance with the vertical synchronization signal from the CCU 421R, for example, the image-capturing unit 401R starts processing of capturing a right-eye image with a 4K resolution and a frame rate of 60P at the timing with a 1/2 frame-cycle difference from the timing of the image-capturing unit 401. Further, the image-capturing unit 401R starts processing of generating a right-eye image-capturing signal including the captured right-eye image and supplying the signal to the signal generation unit 431.

The signal generation unit 431 starts processing of generating a frame-sequential 3D image with a 4K resolution and a frame rate of 120P by alternately arranging the left-eye image included in the left-eye image-capturing signal and the right-eye image included in the right-eye image-capturing signal for each frame. Further, the signal generation unit 431 starts processing of generating a 3D image signal including the generated 3D image and outputting the 3D image signal to each display device 313.

Each display device 313 starts processing of displaying the 3D image based on the 3D image signal.

Thereafter, the image-capturing-timing setting processing ends.

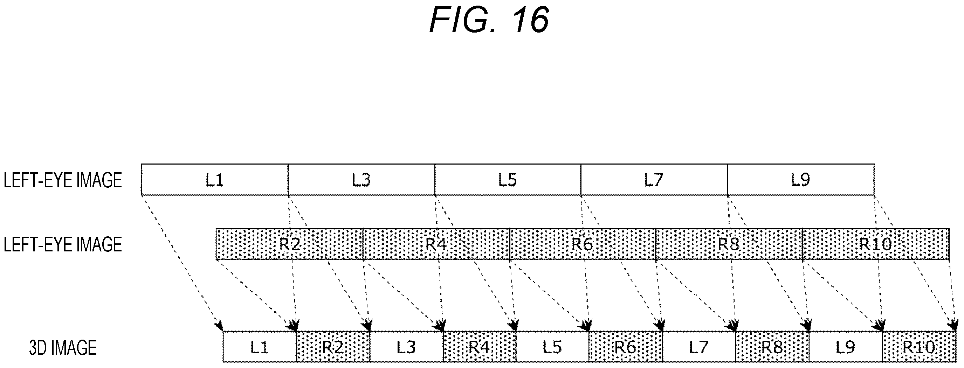

Fig. 16 is a timing chart illustrating the image-capturing timing of a left-eye image, the image-capturing timing of a right-eye image, and the generation timing of a 3D image in a case where the main display device 313 is of the active-retarder type. Here, L represents the left-eye image, R represents the right-eye image, and the respective numbers attached to L and R indicate sequence numbers. Each sequence number indicates an image-capturing order of the corresponding image.

For example, first, capturing of a left-eye image L1 starts, and capturing of a right-eye image R2 starts after the elapse of a 1/2 frame cycle. Thereafter, the subsequent left-eye image L and right-eye image R are captured with a 1/2 frame-cycle difference in timing therebetween.

Further, the image-capturing unit 401L starts output of the left-eye image L1 to the signal generation unit 431 after the duration of about a 1/2 frame cycle elapses from the start of capturing of the left-eye image L1. The output of the left-eye image L1 is performed during about a 1/2 frame cycle, and is completed almost at the same time as the completion of the capturing of the left-eye image L1.

On the other hand, the image-capturing unit 401R starts output of the right-eye image R2 to the signal generation unit 431 after the duration of about a 1/2 frame cycle elapses from the start of capturing of the right-eye image R2. The output of the right-eye image R2 is performed during about a 1/2 frame cycle, and is completed almost at the same time as the completion of the capturing of the right-eye image R2.

Thus, left-eye images L and right-eye images R are alternately output to the signal generation unit 431 at 1/2 frame intervals.

The signal generation unit 431 generates a frame-sequential 3D image by chronologically arranging, in the order of acquisition, the left-eye images L and the right-eye images R alternately supplied at 1/2 frame intervals. The signal generation unit 431 outputs a 3D image signal including the generated 3D image to each display device 313.

In such as manner, a 1/2 frame cycle difference is made between the respective image-capturing timings of each left-eye image L and the corresponding right-eye image R, so that the positional relationship of a subject within each image at the timing of display of each left-eye image L and each right-eye image R is substantially equal to the actual positional relationship. Thus, occurrence of the difference in depth is suppressed, resulting in improvement in the image quality of a 3D image.

Referring back to Fig. 15, otherwise, in a case where it is determined in step S2 that the main display device 313 is not of the active-retarder type, that is, in a case where it is determined that the main display device 313 is of the patterned-retarder type, the processing proceeds to step S4.

In step S4, the microsurgery system 301 synchronizes the respective image-capturing timings of the left and right images.

Specifically, the signal generation unit 431 starts processing of generating a reference clock signal of 60 Hz and supplying the reference clock signal to the image-capturing control unit 432, for example.

The image-capturing control unit 432 starts processing of generating a left-image-capturing control signal and a right-image-capturing control signal on the basis of the reference clock signal, supplying the left-image-capturing control signal to the CCU 421L, and supplying the right-image-capturing control signal to the CCU 421R.

As described above, the left-image-capturing control signal and the right-image-capturing control signal include, respectively, information indicating the timing at which the CCU 421L outputs the vertical synchronization signal and the information indicating the timing at which the CCU 421R outputs the vertical synchronization signal. Then, the image-capturing control unit 432 performs control such that the timing at which the vertical synchronization signal is output from the CCU 421L and the timing at which the vertical synchronization signal is output from the CCU 421R are in synchronization.

For example, the CCU 421L starts processing of generating a vertical synchronization signal of 60 Hz and outputting the signal to the image-capturing unit 401L. In addition, in synchronization with the CCU 421L, the CCU 421R starts processing of generating a vertical synchronization signal of 60 Hz and outputting the signal to the image-capturing unit 401R.

In accordance with the vertical synchronization signal from the CCU 421L, the image-capturing unit 401L starts processing of capturing a left-eye image with a 4K resolution and a frame rate of 60P, for example.
Further, the image-capturing unit 401L starts processing of generating a left-eye image-capturing signal including the captured left-eye image and supplying the signal to the signal generation unit 431.

In synchronization with the image-capturing unit 401 in accordance with the vertical synchronization signal from the CCU 421R, the image-capturing unit 401R starts processing of capturing a right-eye image with a 4K resolution and a frame rate of 60P, for example. Further, the image-capturing unit 401R starts processing of generating a right-eye image-capturing signal including the captured right-eye image and supplying the right-eye image-capturing signal to the signal generation unit 431.

The signal generation unit 431 starts processing of generating a line-by-line 3D image by alternately arranging, for each pixel row, the pixel rows of the left-eye image included in the left-eye image-capturing signal and the pixel rows of the right-eye image included in the right-eye image-capturing signal. Further, the signal generation unit 431 starts processing of generating a 3D image signal including the generated 3D image and outputting the 3D image signal to each display device 313.

Each display device 313 starts processing of displaying a 3D image based on the 3D image signal.

Thereafter, the image-capturing-timing setting processing ends.

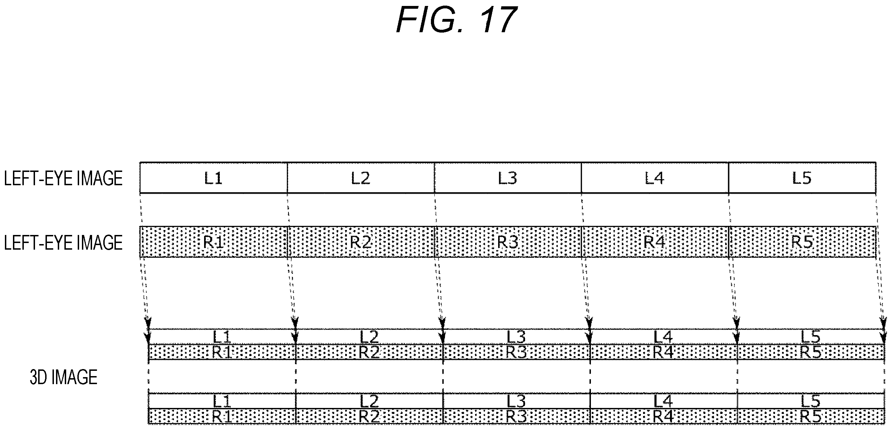

Fig. 17 is a timing chart illustrating the image-capturing timing of a left-eye image, the image-capturing timing of a right-eye image, and the generation timing of a 3D image in a case where the main display device 313 is of the patterned-retarder type. Here, L represents the left-eye image, R represents the right-eye image, and the respective numbers attached to L and R indicate sequence numbers. Each sequence number indicates an image-capturing order of the corresponding image.

For example, capturing of a left-eye image L1 and capturing of a right-eye image R1 simultaneously start, and hereafter, the respective frames of the left-eye image L and the right-eye image R in synchronization are captured at the same timing.

Further, the image-capturing unit 401L and the image-capturing unit 401R synchronously output, respectively, the left-eye image L and the right-eye image R to the signal generation unit 431.

The signal generation unit 431 generates a line-by-line 3D image in which the pixel rows of the left-eye image L and the pixel rows of the right-eye image R are alternately arranged for each pixel row. The signal generation unit 431 outputs a 3D image signal including the generated 3D image to each display device 313.

Thereafter, the image-capturing-timing setting processing ends.

In such a manner, occurrence of a difference in depth in the case of using the active-retarder display device 313 can be suppressed, resulting in improvement in image quality of a 3D image.

In addition, in the case of using the active-retarder display device 313, the resolution in the vertical direction of a 3D image is improved as compared with the case of using the patterned-retarder display device 313. Furthermore, due to no limitation on the vertical field-of-view angle described above with reference to Fig. 2, the definition of an image can be increased by an increase in the resolution of a display device 313 and the display device 313 can be downsized.

Still furthermore, the compatibility with a system used at a conventional surgical location can be secured. For example, used can be a piece of inexpensive and lightweight polarization eyewear 102 typically used in a conventional surgical location. In addition, for example, used can be a patterned-retarder display device 313 used at a conventional surgical location.

### <<3. Modifications>>

Hereinafter, modifications of the above embodiments of the present technology will be described.

The configuration of the microsurgery system 301 in Figs. 13 and 14 is an example of the present technology and thus can be changed.

For example, the image-capturing unit 401L and the image-capturing unit 401R may be provided in image-capturing devices having different casings, respectively.

For example, the CCU 421L and the CCU 421R may be provided separately from the control device 312, or the CCU 421L and the CCU 421R may be integrated.

For example, the image-capturing control unit 432 may detect the display type of each display device 313.

In addition, in the above description, an example has been given in which the main display device 313 for use in control of each image-capturing timing is set as the display device 313 viewed by the main surgeon. The main display device 313, however, may be set on the basis of different conditions. For example, the display device 313 viewed by the largest number of users may be set as the main display device 313.

Further, for example, each image-capturing timing may be controlled on the basis of the most frequent display type among the plurality of display devices 313.

In addition, the present technology is applicable to the video microscopic surgery described above, and to, for example, a 3D surgical system for various types of surgery with 3D images, such as video endoscopic surgery and open imaging surgery (video laparotomy).

Further, the present technology is also applicable to a case of capturing and displaying a 3D image for use in applications different from surgery.

### <<4. Others>>

### <Exemplary Configuration of Computer>

The series of processing described above (for example, processing of the control device 312) can be executed with hardware or software. In a case where the series of processing is performed with software, a program included in the software is installed on a computer. Here, examples of the computer include a computer embedded in dedicated hardware, a general-purpose personal computer or the like capable of executing various functions by installation of various programs, and the like.

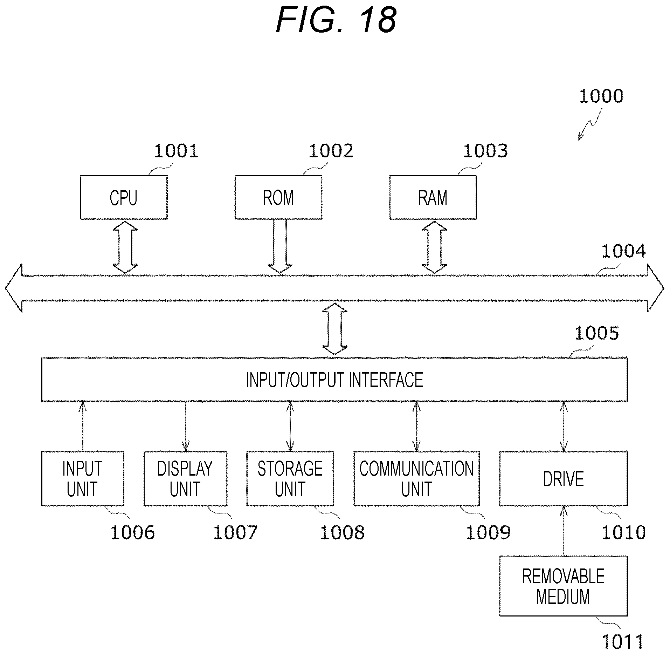

Fig. 18 is a block diagram of an exemplary hardware configuration of a computer that uses a program to execute the series of processing described above.

In the computer, a central processing unit (CPU) 1001, a read only memory (ROM) 1002, and a random access memory (RAM) 1003 are mutually connected through a bus 1004.

Further, an input/output interface 1005 is connected to the bus 1004. An input unit 1006, an output unit 1007, a storage unit 1008, a communication unit 1009, and a drive 1010 are connected to the input/output interface 1005.

The input unit 1006 includes a keyboard, a mouse, a microphone, and the like. The output unit 1007 includes a display, a speaker, and the like. The storage unit 1008 includes a hard disk, a non-volatile memory, or the like. The communication unit 1009 includes a network interface or the like. The drive 1010 drives a removable medium 1011 such as a magnetic disk, an optical disk, a magneto-optical disk, or a semiconductor memory.

In the computer having the configuration as above, the CPU 1001 loads, for example, a program stored in the storage unit 1008, into the RAM 1003 through the input/output interface 1005 and the bus 1004 to execute the program, so that the series of processing described above is performed.

The program executed by the computer (CPU 1001) can be provided by being recorded on, for example, the removable medium 1011 as a package medium or the like. Alternatively, the program can be provided through a wired or wireless transmission medium such as a local area network, the Internet, or digital satellite broadcasting.

In the computer, the program can be installed in the storage unit 1008 through the input/output interface 1005 by attachment of the removable medium 1011 to the drive 1010. Alternatively, the program can be received by the communication unit 1009 through the wired or wireless transmission medium and can be installed in the storage unit 1008. In addition, the program can be preinstalled in the ROM 1002 or the storage unit 1008.

Note that the program executed by the computer may be a program for chronologically performing the processing in accordance with the order described in the present description, may be a program for parallelly performing the processing, or a program for performing the processing with necessary timing, for example, when a call is made.

Further, in the present description, the system means a collection of a plurality of constituent elements (devices, modules (components), and others). Thus, it is not concerned whether or not all the constituent elements are included in the same casing. Therefore, a plurality of devices housed in separate casings and connected through a network, and a single device having a plurality of modules housed in a single casing are both systems.

Note that embodiments of the present technology are not limited to the above embodiments, and thus various modifications can be made within the scope without departing from the gist of the present technology.

For example, the present technology can adopt a cloud computing configuration in which a single function is shared and processed by a plurality of devices through a network.

In addition, each step described in the above flowchart can be performed by a single device, or can be performed by sharing among a plurality of devices.

Furthermore, in a case where a plurality of pieces of processing is included in a single step, the plurality of pieces of processing included in the single step can be performed by a single device, or can be performed by sharing among a plurality of devices.

### <Exemplary Configuration Combinations>

The present technology can also adopt the following configurations.

(1)
   A surgical image-capturing system including:
   a first image-capturing unit configured to capture a surgical site and output a first image-capturing signal;
   a second image-capturing unit configured to capture the surgical site at an angle different from an angle of the first image-capturing unit in a frame cycle identical to a frame cycle of the first image-capturing unit and output a second image-capturing signal;
   an image-capturing control unit configured to control respective image-capturing timings of the first image-capturing unit and the second image-capturing unit; and
   a signal generation unit configured to generate a three-dimensional (3D) image signal on the basis of the first image-capturing signal and the second image-capturing signal,
   in which the image-capturing control unit performs control such that a difference is made by half the frame cycle between the respective image-capturing timings of the first image-capturing unit and the second image-capturing unit.
(2)
   The surgical image-capturing system according to (1) described above,
   in which the image-capturing control unit controls the respective image-capturing timings of the first image-capturing unit and the second image-capturing unit on the basis of a display type of a display device that displays a 3D image based on the 3D image signal.
(3)
   The surgical image-capturing system according to (2) described above,
   in which in a case where the display device is of an active-retarder type, the image-capturing control unit performs control such that a difference is made by half the frame cycle between the respective image-capturing timings of the first image-capturing unit and the second image-capturing unit, and in a case where the display device is of a patterned-retarder type, the image-capturing control unit performs control such that the respective image-capturing timings of the first image-capturing unit and the second image-capturing unit are in synchronization.
(4)
   The surgical image-capturing system according to (3) described above,
   in which in the case where the display device is of the active-retarder type, the signal generation unit generates the 3D image signal of a frame-sequential type on the basis of the first image-capturing signal and the second image-capturing signal, and in the case where the display device is of the patterned-retarder type, the signal generation unit generates the 3D image signal of a line-by-line type on the basis of the first image-capturing signal and the second image-capturing signal.
(5)
   The surgical image-capturing system according to any of (2) to (4) described above,
   in which the image-capturing control unit controls the respective image-capturing timings of the first image-capturing unit and the second image-capturing unit on the basis of a signal indicating the display type from the display device.
(6)
   The surgical image-capturing system according to any of (2) to (5) described above,
   in which in a case where a plurality of the display devices that displays the 3D image is provided, the image-capturing control unit controls the respective image-capturing timings of the first image-capturing unit and the second image-capturing unit on the basis of the display type of the display device for a main surgeon.
(7)
   The surgical image-capturing system according to (1) described above,
   in which the signal generation unit outputs the 3D image signal to a display device of an active-retarder type.
(8)
   The surgical image-capturing system according to (7) described above,
   in which the signal generation unit generates the 3D image signal of a frame-sequential type on the basis of the first image-capturing signal and the second image-capturing signal.
(9)
   The surgical image-capturing system according to any of (1) to (8) described above,
   in which the first image-capturing unit and the second image-capturing unit each perform image-capturing at a 4K resolution and at a frame rate of 60P, and
   the signal generation unit generates the 3D image signal with a 4K resolution and a frame rate of 120P.
(10)
   A signal processing device including:
   an image-capturing control unit configured to control respective image-capturing timings of a first image-capturing unit and a second image-capturing unit, the first image-capturing unit being configured to capture a surgical site and output a first image-capturing signal, the second image-capturing unit being configured to capture the surgical site at an angle different from an angle of the first image-capturing unit in a frame cycle identical to a frame cycle of the first image-capturing unit and output a second image-capturing signal; and
   a signal generation unit configured to generate a 3D image signal on the basis of the first image-capturing signal and the second image-capturing signal,
   in which the image-capturing control unit performs control such that a difference is made by half the frame cycle between the respective image-capturing timings of the first image-capturing unit and the second image-capturing unit.
(11)
   A signal processing method including:
   performing control such that a difference is made by half a frame cycle between respective image-capturing timings of a first image-capturing unit and a second image-capturing unit, the first image-capturing unit being configured to capture a surgical site and output a first image-capturing signal, the second image-capturing unit being configured to capture the surgical site at an angle different from an angle of the first image-capturing unit in the frame cycle identical to the frame cycle of the first image-capturing unit and output a second image-capturing signal; and
   generating a 3D image signal on the basis of the first image-capturing signal and the second image-capturing signal.

Note that the effects described in the present description are merely exemplified and are not intended to be limiting; thus, there may have additional effects.

### REFERENCE SIGNS LIST

- 100: 3D display system
- 101: Display device
- 102: Polarization eyewear
- 111: Display panel
- 112: Polarizing plate
- 113: Patterned Retarder
- 200: 3D display system
- 201: Display device
- 211: Display panel
- 212: Polarizing plate
- 213: Active retarder
- 301: Microsurgery system
- 311: Microscope device
- 312: Control device
- 313-1: to 313-n Display device
- 401L,: 401R Image-capturing unit
- 421L,: 421R CCU
- 422: 3D signal synthesizer
- 431: Signal generation unit
- 432: Image-capturing control unit

## Claims

1. A surgical image-capturing system comprising:
a first image-capturing unit configured to capture a surgical site and output a first image-capturing signal;
a second image-capturing unit configured to capture the surgical site at an angle different from an angle of the first image-capturing unit in a frame cycle identical to a frame cycle of the first image-capturing unit and output a second image-capturing signal;
an image-capturing control unit configured to control respective image-capturing timings of the first image-capturing unit and the second image-capturing unit; and
a signal generation unit configured to generate a three-dimensional (3D) image signal on a basis of the first image-capturing signal and the second image-capturing signal,
wherein the image-capturing control unit performs control such that a difference is made by half the frame cycle between the respective image-capturing timings of the first image-capturing unit and the second image-capturing unit.

2. The surgical image-capturing system according to claim 1,
wherein the image-capturing control unit controls the respective image-capturing timings of the first image-capturing unit and the second image-capturing unit on a basis of a display type of a display device that displays a 3D image based on the 3D image signal.

3. The surgical image-capturing system according to claim 2,
wherein in a case where the display device is of an active-retarder type, the image-capturing control unit performs control such that a difference is made by half the frame cycle between the respective image-capturing timings of the first image-capturing unit and the second image-capturing unit, and in a case where the display device is of a patterned-retarder type, the image-capturing control unit performs control such that the respective image-capturing timings of the first image-capturing unit and the second image-capturing unit are in synchronization.

4. The surgical image-capturing system according to claim 3,
wherein in the case where the display device is of the active-retarder type, the signal generation unit generates the 3D image signal of a frame-sequential type on a basis of the first image-capturing signal and the second image-capturing signal, and in the case where the display device is of the patterned-retarder type, the signal generation unit generates the 3D image signal of a line-by-line type on a basis of the first image-capturing signal and the second image-capturing signal.

5. The surgical image-capturing system according to claim 2,
wherein the image-capturing control unit controls the respective image-capturing timings of the first image-capturing unit and the second image-capturing unit on a basis of a signal indicating the display type from the display device.

6. The surgical image-capturing system according to claim 2,
wherein in a case where a plurality of the display devices that displays the 3D image is provided, the image-capturing control unit controls the respective image-capturing timings of the first image-capturing unit and the second image-capturing unit on a basis of the display type of the display device for a main surgeon.

7. The surgical image-capturing system according to claim 1,
wherein the signal generation unit outputs the 3D image signal to a display device of an active-retarder type.

8. The surgical image-capturing system according to claim 7,
wherein the signal generation unit generates the 3D image signal of a frame-sequential type on a basis of the first image-capturing signal and the second image-capturing signal.

9. The surgical image-capturing system according to claim 1,
wherein the first image-capturing unit and the second image-capturing unit each perform image-capturing at a 4K resolution and at a frame rate of 60P, and
the signal generation unit generates the 3D image signal with a 4K resolution and a frame rate of 120P.

10. A signal processing device comprising:
an image-capturing control unit configured to control respective image-capturing timings of a first image-capturing unit and a second image-capturing unit, the first image-capturing unit being configured to capture a surgical site and output a first image-capturing signal, the second image-capturing unit being configured to capture the surgical site at an angle different from an angle of the first image-capturing unit in a frame cycle identical to a frame cycle of the first image-capturing unit and output a second image-capturing signal; and
a signal generation unit configured to generate a 3D image signal on a basis of the first image-capturing signal and the second image-capturing signal,
wherein the image-capturing control unit performs control such that a difference is made by half the frame cycle between the respective image-capturing timings of the first image-capturing unit and the second image-capturing unit.

11. A signal processing method comprising:
performing control such that a difference is made by half a frame cycle between respective image-capturing timings of a first image-capturing unit and a second image-capturing unit, the first image-capturing unit being configured to capture a surgical site and output a first image-capturing signal, the second image-capturing unit being configured to capture the surgical site at an angle different from an angle of the first image-capturing unit in the frame cycle identical to the frame cycle of the first image-capturing unit and output a second image-capturing signal; and
generating a 3D image signal on a basis of the first image-capturing signal and the second image-capturing signal.
